# FASCICULE DE BREVET EUROPEEN

(11) **EP 0 754 026 B1**
(45) Date de publication et mention de la délivrance du brevet: **03.11.1999**
(21) Numéro de dépôt: 96901837.3
(22) Date de dépôt: 23.01.1996
(51) Int. Cl.: A61K 7/06

(54) **COMPOSITION COSMETIQUE A BASE DE GOMME DE GUAR NON IONIQUE ET DE POLYMERE ANIONIQUE NON RETICULE**
KOSMETISCHE ZUSAMMENSETZUNG AUF DER BASIS VON NICHTIONISCHEM GUARGUMMI UND NICHT VERNETZTEM ANIONISCHEN POLYMER
COSMETIC COMPOSITION CONTAINING NON-IONIC GUAR GUM AND A NON-CROSS LINKED ANIONIC POLYMER

(30) Priorité: 02.02.1995 FR 9501228
(43) Date de publication de la demande: 22.01.1997
(73) Titulaire: L'OREAL, 75008 Paris (FR)
(72) Inventeur: SAMAIN, Henri, F-91570 Bièvres (FR); CRETOIS, Isabelle, F-92110 Clichy (FR)
(74) Mandataire: Tezier Herman, Béatrice
(86) Numéro de dépôt international: FR9600110
(87) Numéro de publication internationale: WO9623482

(56) Documents cités:
- EP-A- 0 152 095
- EP-A- 0 320 218
- EP-A- 0 412 705
- WO-A-92/21316
- FR-A- 2 670 673
- GB-A- 2 136 689
- CHEMICAL ABSTRACTS, vol. 120, no. 18, 2 Mai 1994 Columbus, Ohio, US; abstract no. 226526, YOSHIO I. ET AL: "Hair-setting aerosols containing film-forming polymers and guar gums " XP002002182 & PATENT ABSTRACTS OF JAPAN vol. 18, no. 191 (C-1186), 4 Avril 1994 & JP,A,05 345708 (KAO CORP.), 27 Décembre 1993, & DATABASE WPI Section Ch, Week 945 Derwent Publications Ltd., London, GB; Class D08, AN 94-040015 & JP,A,05 345 708 (KAO CORP) , 27 Décembre 1993

## Description

La présente invention concerne des compositions cosmétiques non lavantes contenant, dans un milieu cosmétiquement acceptable, au moins une gomme de guar non ionique et au moins un polymère anionique non réticulé.
L'invention a encore pour objet un procédé de traitement cosmétique non lavant des matières kératiniques, telles que les cheveux ou les cils, caractérisé en ce qu'il consiste à appliquer sur les matières kératiniques une composition cosmétique telle que définie précédemment, puis à effectuer éventuellement un rinçage à l'eau.

On connaît déjà dans l'état de la technique des formulations permettant de fixer et de conditionner les cheveux. On a déjà utilisé dans ce but des compositions sous forme de gel qui sont généralement à base de polymères acryliques réticulés. Ces compositions présentent toutefois l'inconvénient de laisser un dépôt indésirable sur les cheveux qui nuit aux propriétés cosmétiques de ces derniers : ainsi, en fin d'opération, les cheveux sont peu brillants, rêches ou collants.

On a également associé des polymères anioniques connus pour apporter du maintien et/ou de la fixation à la chevelure avec des polymères cationiques en particulier des gommes de guar cationiques. Cependant, la demanderesse a constaté en particulier que les cheveux avaient un toucher désagréable et que la tenue de la coiffure était faible.
De plus, les produits à base de polymères anioniques présentent souvent des problèmes liés à la texture du produit. En effet, il est difficile d'obtenir des produits s'étalant bien sur les cheveux.

Le brevet GB-A-2 136 689 décrit l'utilisation de gomme de xanthan et d'au moins un polymère anionique dans un après-shampoing, afin d'améliorer la stabilité et l'homogénéité de la composition. Une gomme de guar (ionique ou non) peut être ajoutée pour en modifier la consistance.

Les gommes de guar non ioniques sont décrites comme étant des agents épaississants.

Or, la demanderesse vient maintenant de découvrir que l'association de gommes de guar non ioniques avec des polymères anioniques non réticulés conduisait de façon inattendue et surprenante à des propriétés particulièrement intéressantes, notamment à une amélioration de la tenue de la coiffure dans le temps et du pouvoir fixant. La chevelure a plus de volume, les cheveux sont brillants, et présentent un toucher naturel.

De plus, ces compositions s'étalent plus facilement sur les cheveux et la texture de la composition est plus agréable.

Cette découverte est à la base de la présente invention.

La présente invention a donc pour objet de nouvelles compositions cosmétiques non lavantes des matières kératiniques telles que les cheveux, la peau ou les cils, contenant dans un milieu cosmétiquement acceptable non détergent, au moins une gomme de guar non ionique et au moins un polymère anionique non réticulé choisi parmi :
A) - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters ;
   - les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupement acrylamide, méthacrylamide, α-oléfine, esters acryliques ou méthacryliques, acides acrylique ou méthacrylique ou vinylpyrrolidone dans leur chaîne,
   les fonctions anhydrides de ces copolymères étant éventuellement monoestérifiées ou monoamidifiées.
(B) - les copolymères des acides acrylique ou méthacrylique avec un ou plusieurs monomères monoéthyléniques tels que l'éthyléne, le styrène, les esters vinyliques, les esters d'acide acrylique ou méthacrylique.
(C) - les copolymères comprenant (i) un ou plusieurs acides acrylique ou méthacrylique, (ii) un ou plusieurs monomères monoéthyléniques tels que l'éthylène, le styrène, les esters vinyliques, les esters d'acide acrylique ou méthacrylique et (iii)-un ou plusieurs monomères choisis parmi les vinyllactames, les éthers d'allyle ou méthallyle et d'alkyle en C6-C22 éventuellement polyoxyalkylénés, l'acétate de vinyle et les N-alkyl(C3-C10)acrylamides.

D'autres caractéristiques, aspects, objets et avantages de l'invention apparaîtront encore plus clairement à la lecture de la description et des exemples qui suivent.

Par compositions non lavantes, on désigne selon l'invention des compositions n'ayant pas un caractère détergent. De préférence, les compositions contiennent moins de 4% en poids de tensioactifs détergents.

Selon l'invention, on peut utiliser les gommes de guar non ioniques chimiquement modifiées ou non modifiées.

Les gommes de guar non ioniques non modifiées sont par exemple les produits vendu; sous la dénomination VIDOGUM GH 175 par la société UNIPECTINE et sous la dénomination JAGUAR C par la société MEYHALL.

Les gommes de guar non-ioniques utilisables selon l'invention sont de préférence modifiées par des groupements hydroxyalkyle en C₁-C₆.

Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2 et.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société RHONE POULENC (MEYHALL) ou sous la dénomination GALACTASOL 4H4FD2 par la société AQUALON.

Selon l'invention, on peut utiliser les polymères anioniques comportant des groupements dérivés d'acide carboxylique ayant de préférence un poids moléculaire compris entre environ 500 et 5.000.000.

Les polymères du groupe (A) sont décrits en particulier dans les brevets US 2.047.398, 2.723.248, 2.102.113, le brevet GB 839.805 et sont notamment les produits vendus sous les dénominations GANTREZ AN ou ES par la société ISP. Des polymères du groupe (A) sont également par exemple décrits dans les brevets français 2.350.384 et 2.357.241 de la demanderesse. L'enseignement de ces brevets est inclus dans la présente demande.

Les polymères du groupe (B) sont décrits en particulier dans le brevet français 1.222.944 et la demande allemande 2.330.956.
Les esters d'acide acrylique ou méthacrylique sont par exemple les acrylates ou méthacrylates d'alkyle en C₁-C₂₀.

Parmi les polymères du groupe (B), on préfère les copolymères d'acide méthacrylique et d'acrylate d'alkyle en C₁-C₆ , les copolymères d'acide acrylique ou méthacrylique, d'acrylate ou de méthacrylate d'alkyle et de styrène.

Dans les copolymères du groupe (C), les esters d'acide acrylique ou méthacrylique sont par exemple les acrylates ou méthacrylates d'alkyle en C₁-C₂₀.
Parmi les monomères (iii), les vinyllactames sont par exemple la vinylpyrrolidone ou le vinylcaprolactame. Les éthers d'allyle ou de méthallyle sont par exemple les allyléther d'alkyle en C12-C22 polyoxyalkyléné.
Le nombre de groupements oxyde d'alkylène peut varier de 2 à 100. Les oxyde d'alkylène ont de préférence 2 ou 3 atomes de carbone.
Parmi les monomères (iii), on préfère plus particulièrement l'allyl éther de stéaryl polyoxyéthyléné et la vinylpyrrolidone.
Les copolymères du groupe (C) sont par exemple, les copolymères d'acide acrylique ou méthacrylique, d'acrylate ou de méthacrylate d'alkyle et de N-tertiobutylacrylamide, les terpolymères de vinylpyrrolidone/acide acrylique/méthacrylate de lauryle, les terpolymère d'acide méthacrylique, d'acrylate d'éthyle et d'allyl éther de stéaryl polyoxyéthyléné à 10 moles d'oxyde d'éthyléne.

Selon l'invention, les polymères anioniques du groupe (A) sont de préférence choisis parmi les copolymères comprenant (i) des acides ou d'anhydrides maléique, fumarique, itaconique , (ii) des esters vinyliques, des éthers vinyliques, des halogénures vinyliques, des dérivés phénylvinyliques, l'acide acrylique et ses esters, tels que les copolymères méthylvinyléther/anhydride maléique mono estérifié par des alcools en C₁-C₆ vendus sous les dénominations GANTREZ par la société ISP.

On utilise de préférence les copolymères méthylvinyléther/anhydride maléique mono estérifié par butanol vendu; sous la dénomination GANTREZ ES 425 par la société ISP,les copolymères d'acide méthacrylique et d'acrylate d'alkyle en C₁-C₆ tels que l'acrylate d'éthyle vendus par exemple sous la dénomination LUVIMER MAE ou MAEX par la société BASF, les terpolymères de vinylpyrrolidone/acide acrylique/méthacrylate de lauryle vendus par exemple sous la dénomination ACRYLIDONE LM par la société ISP.

La concentration en gomme de guar non ioniques peut varier entre 0,01 % et 5 % en poids environ par rapport au poids total de la composition, et de préférence entre 0,1 et 2,5 % environ.

La concentration en polymère anionique non réticulé peut varier entre 0,01 et 8 % en poids environ par rapport au poids total de la composition, et de préférence entre 0,1 et 3 % environ.

Le rapport pondéral gomme de guar non ionique/polymère anionique non réticulé est de préférence inférieur à 1.

Le milieu cosmétiquement acceptable est de préférence constitué par de l'eau ou un mélange d'eau et de solvants cosmétiquement acceptables tels que des monoalcools, des polyalcools, des éthers de glycol ou des esters d'acide; gras, qui peuvent être utilisés seuls ou en mélange.

On peut citer plus particulièrement les alcools inférieurs tels que l'éthanol, l'isopropanol, les polyalcools tels que le diéthylèneglycol, les éthers de glycol, les alkyléthers de glycol ou de diéthylèneglycol.

Le pH des compositions selon l'invention est généralement compris entre 2 et 9, et en particulier entre 3 et 8. Il peut être ajusté à la valeur choisie au moyen d'agents alcalinisants ou acidifiants habituellement utilisés en cosmétique pour ce type d'application.

Les compositions selon l'invention peuvent encore contenir, des agents épaississants, des agents tensioactifs, des agents conservateurs, des séquestrants, des adoucissants, des parfums, des colorants, des agents modificateurs de viscosité, des agents modificateurs de mousse, des agents moussants, des stabilisateurs de mousse, des agents nacrants, des agents hydratants, des agents antipelliculaires, des agents antiséborrhéiques, des filtres solaires, des protéines, des vitamines, des plastifiants, des hydroxyacides, des électrolytes, des agents propulseurs et des parfums.

Les compositions selon l'invention peuvent également contenir des agents conditionneurs. Ceux-ci peuvent alors être choisis parmi les huiles et les cires naturelles ou synthétiques, les alcools gras, les esters d'alcools polyhydriques, les glycérides, les huiles, les gommes et résines de silicone ou les mélanges de ces différents composés.

L'invention a encore pour objet un procédé de traitement de la peau ou des fibres kératiniques, telles que les cheveux ou les cils, caractérisé en ce qu'il consiste à appliquer sur la peau ou sur les fibres kératiniques une composition cosmétique telle que définie précédemment, puis à effectuer éventuellement un rinçage à l'eau, après un éventuel temps de pose.

Ainsi, ce procédé selon l'invention permet le maintien de la coiffure ou des cils, le traitement, le soin de la peau, des cheveux ou de toute autre matière kératinique.

Les compositions cosmétiques selon l'invention peuvent se présenter sous forme de gel, de lait, de crème, de gel-crème, de spray, de lotion plus ou moins épaissie ou de mousse et être utilisées par exemple pour la peau, les cheveux , les cils ou les sourcils.

Pour les cheveux, elles sont plus particulièrement des compositions à rincer ou non, à appliquer avant ou après un shampooing, une coloration, une décoloration, une permanente ou un défrisage.

Les compositions peuvent être également des lotions de mise en plis, des lotions pour le brushing, des compositions de fixation et de coiffage.

Les lotions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, les hydrocarbures chlorés et/ou fluorés et leurs mélanges. On peut également utiliser en tant qu'agent propulseur le gaz carbonique, le protoxyde d'azote, le diméthyléther, l'azote ou l'air comprimé.

Dans tout ce qui suit ou ce qui précède, les pourcentages exprimés sont en poids.

L'invention va être maintenant plus complètement illustrée à l'aide des exemples suivants.

Dans les exemples, MA signifie matière active.

### EXEMPLE 1

On a préparé un gel (1), conforme à l'invention, de composition suivante :

| | |
|---|---|
| - Gomme de guar non-ionique modifiée par des groupements hydroxypropyle vendue sous la dénomination commerciale JAGUAR HP60 par la société MEYHALL | 0,95 g |
| - Copolymère méthylvinyléther/monomaléate de butanol neutralisé à 100% par de l'AMP (GANTREZ ES 425 de ISP) | 2 g MA |
| - Eau qsp | 100 g |

On a préparé un gel (2), comparatif, de composition suivante :

| | |
|---|---|
| - Gomme de guar cationique vendue sous la dénomination commerciale JAGUAR C13S par la société MEYHALL | 0,95 g |
| - GANTREZ ES 425 neutralisé à 100% par de l'AMP(ISP) | 2 gMA |
| - Eau qsp | 100 g |

On a préparé un gel (3), comparatif, de composition suivante :

| | |
|---|---|
| - Polymère d'acide acrylique réticulé vendu sous la dénomination commerciale CARBOPOL 940 par la société GOODRICH | 0,95 g |
| - GANTREZ ES 425 (ISP) | 2 g |
| - Monoéthanolamine qs | pH 7 |
| - Eau déminéralisée q.s.p. | 100 g |

On a préparé un gel (4), comparatif, de composition suivante :

| | |
|---|---|
| - Copolymère acétate de vinyle/vinylpyrrolidone vendu sous la dénomination commerciale LUVISKOL VA 64 par la société BASF | 0,95 g |
| - GANTREZ ES 425 (ISP) neutralisé à 100% par de l'AMP | 2 gMA |
| - Eau qsp | 100 g |

Sur des mèches de cheveux lavés et essorés, on a appliqué 0,5 g de gel par g de cheveux. On a ensuite procédé à un brushing.

On a ensuite demandé à un panel de 8 juges d'évaluer le plixant (durcissement de la fibre) et le toucher des cheveux.

### Notation :

Plixant : 0 (pas de plixant) à 5 (fixation très forte)
Toucher : 0 (très mauvais) à 5 (excellent)

Après 5 heure; à température et humidité ambiante, les mêmes juges ont évalué la tenue de la coiffure.

### Notation :

Tenue : 0 (pas de tenue) à 5 (tenue parfaite)

Les résultats (moyenne des notes) sont rassemblés dans le tableau ci-dessous :

| | **Plixant** | **Toucher** | **Tenue** |
|---|---|---|---|
| **Gel (1) Invention** | 4 | 4 | 4 |
| **Gel (2) Comparatif** | 2,25 | 0,75 | 1,25 |
| **Gel (3) Comparatif** | 2 | 0,75 | 1,5 |
| **Gel (4) Comparatif** | 4 | 0,5 | 2,75 |

Seul le gel 1 selon l'invention présente de très bonnes propriétés de plixant, de toucher et de tenue.

### EXEMPLE 2

On a préparé un gel selon l'invention de composition suivante :

| | |
|---|---|
| - Gomme de guar non-ionique modifiée par des groupements hydroxypropyle vendue sous la dénomination commerciale JAGUAR HP60 par la société MEYHALL | 1,45 g |
| - Polymère anionique neutralisé à 100% par de l'AMP | 1,5 gMA |
| - Eau qsp | 100 g |

### Polymère anionique :

Gel (5) : GANTREZ ES 425 (ISP)
Gel (6) :Copolymère acide acrylique/acrylate d'éthyle (LUVIMER de BASF)

Sur des mèches de cheveux lavés et essorés, on a appliqué 0,5 g de gel par g de cheveux. On a ensuite procédé à un brushing.

On a ensuite demandé à un panel de 8 juges d'évaluer le plixant, le toucher et la tenue de la coiffure.

Les résultats (moyenne des notes) sont rassemblés dans le tableau ci-dessous :

| | **Plixant** | **Toucher** | **Tenue** |
|---|---|---|---|
| **Gel (5)** | 4 | 3,75 | 3,75 |
| **Gel (6)** | 4 | 4 | 4 |

Les gels selon l'invention présentent de bonnes propriétés de plixant, de toucher et de tenue

### EXEMPLE 3

On a préparé une mousse de coiffage de composition suivante :

| | |
|---|---|
| - Gomme de guar non-ionique modifiée par des groupements hydroxypropyle vendue sous la dénomination commerciale JAGUAR HP60 par la société MEYHALL | 0,5 g |
| - GANTREZ ES 425 (ISP) neutralisé à 100% par de l'AMP | 2 gMA |
| - LUVISKOL VA 64 (BASF) | 1 g |
| - Alcool éthylique | 10 g |
| - 2-amino 2-méthyl 1-propanol | qs pH7 |
| - Parfum, conservateur qs | |
| - Eau déminéralisée qsp | 100g |

### Conditionnement en aérosol :

90g de la composition ci-dessus sont conditionnés dans un récipient aérosol en présence de 10g d'un mélange ternaire de n-butane, isobutane et propane, (23/55/22), vendu sous la dénomination de "AEROGAZ 3,2 N par la société ELF AQUITAINE

Cette mousse présente une texture crémeuse très agréable.
Cette mousse a été appliquée sur les cheveux mouillés, elle se répartit facilement sur l'ensemble de la chevelure et facilite le démêlage des cheveux.
On procède à un brushing, la coiffure obtenue est gonflante, les cheveux sont brillants, sans résidus et le toucher est naturel.
La coiffure présente une bonne tenue dans le temps.

### EXEMPLE 4

On a préparé une mousse de coiffage de composition suivante :

| | |
|---|---|
| - Gomme de guar non-ionique modifiée par des groupements hydroxypropyle vendue sous la dénomination commerciale JAGUAR HP60 par la société MEYHALL | 0,5 g |
| - LUVIMER (BASF) neutralisé à 100% par de l'AMP | 1 gMA |
| - LUVISKOL VA 64 (BASF) | 0,8 g |
| - Alcool éthylique | 10 g |
| - Triacétine | 0,33 g |
| - 2-amino 2-méthyl 1-propanol | qs pH7 |
| - Parfum, conservateur qs | |
| - Eau déminéralisée qsp | 100g |

### Conditionnement en aérosol :

90g de la composition ci-dessus sont conditionnés dans un récipient aérosol en présence de 10g d'un mélange ternaire de n-butane, isobutane et propane, (23/55/22), vendu sous la dénomination de "AEROGAZ 3,2 N par la société ELF AQUITAINE

Cette mousse présente une texture crémeuse très agréable.
Cette mousse a été appliquée sur les cheveux mouillés, elle se répartit facilement sur l'ensemble de la chevelure et facilite le démêlage des cheveux.
On procède à un brushing, la coiffure obtenue est gonflante, les cheveux sont brillants, sans résidus et le toucher est naturel.
La coiffure présente une bonne tenue dans le temps.

### EXEMPLE 5

On a préparé un gel de composition suivante :

| | |
|---|---|
| - Gomme de guar non-ionique modifiée par des groupements hydroxypropyle vendue sous la dénomination commerciale JAGUAR HP60 par la société MEYHALL | 0,95 g |
| - Polymère anionique neutralisé à 100% par de l'AMP | 1 gMA |
| - Eau qsp | 100 g |

### Polymère anionique :

Gel (7) : GANTREZ ES 425 (ISP)
Gel (8) : Copolymère acide acrylique/acrylate d'éthyle (LUVIMER MAE de BASF)
Gel (9) : Copolymère acrylate de sodium/vinyl carbinol (HYDAGEN FN de HENKEL)

Sur des mèches de cheveux lavés et essorés, on a appliqué 0,5 g de gel par g de cheveux. On a ensuite procédé à un brushing.

On a ensuite demandé à un panel de 8 juges d'évaluer le plixant et le toucher de la coiffure.

Les résultats (moyenne des notes) sont rassemblés dans le tableau ci-dessous :

| | **Plixant** | **Toucher** |
|---|---|---|
| **Gel (7)Invention** | 4 | 4 |
| **Gel (8)Invention** | 3,5 | 2,25 |
| **Gel (9)Comparatif** | 2,5 | 2 |

Les gels selon l'invention (7) et (8) présentent de meilleures propriétés de plixant et de toucher.

### EXEMPLE 6

On a préparé un gel de coiffage de composition suivante :

| | |
|---|---|
| - Gomme de guar non-ionique modifiée par des groupements hydroxypropyle vendue sous la dénomination commerciale JAGUAR HP60 par la société MEYHALL | 0,95 g |
| - Acide méthacrylique/méthacrylate de lauryle/vinylpyrrolidone (ACRYLIDONE LM d'ISP) | 2 gMA |
| - Alcool éthylique | 10 g |
| - 2-amino 2-méthyl 1-propanol | qs pH7 |
| - Parfum, conservateur qs | |
| - Eau déminéralisée qsp | 100g |

Ce gel a été appliqué sur les cheveux mouillés, il se répartit facilement sur l'ensemble de la chevelure et facilite le démêlage des cheveux.
On procède à un brushing, la coiffure obtenue est gonflante, les cheveux sont brillants, sans résidus.
La coiffure présente une bonne tenue dans le temps.

## Revendications

1. Composition cosmétique non lavante, caractérisée par le fait qu'elle contient, dans un milieu cosmétiquement acceptable, au moins une gomme de guar non ionique et au moins un polymère anionique non réticulé choisi parmi :
A) - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters ;
- les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupement acrylamide, méthacrylamide, α-oléfine, esters acryliques ou méthacryliques, acides acrylique ou méthacrylique ou vinylpyrrolidone dans leur chaîne,
les fonctions anhydrides de ces copolymères étant éventuellement monoestérifiées ou monoamidifiées.
(B) - les copolymères des acides acrylique ou méthacrylique avec un ou plusieurs monomères monoéthyléniques tels que l'éthylène, le styrène, les esters vinyliques, les esters d'acide acrylique ou méthacrylique,
(C) - les copolymères comprenant (i) un ou plusieurs acides acrylique ou méthacrylique, (ii) un ou plusieurs monomères monoéthyléniques tels que l'éthyléne, le styrène, les esters vinyliques, les esters d'acide acrylique ou méthacrylique et (iii) un ou plusieurs monomères choisis parmi les vinyllactames, les éthers d'allyle ou méthallyle et d'alkyle en C6-C22 éventuellement polyoxyalkylénés, l'acétate de vinyle et les N-alkyl(C3-C10)acrylamides.

2. Composition selon la revendication 1, caractérisée par le fait que la gomme de guar non-ionique est modifiée par des groupements hydroxylakyle en C₁-C₆.

3. Composition selon la revendication 2 caractérisée par le fait que les groupements hydroxyalkyle sont choisis parmi les groupements hydroxyméthyle, hydroxyéthyle, hydroxypropyle et hydroxybutyle.

4. Composition selon l'une quelconque des revendications 2 et 3, caractérisée par le fait que la gomme de guar non-ionique présente un taux d'hydroxyalkylation variant entre 0,4 et 1,2.

5. Composition selon l'une quelconque des revendications précédentes, caractérisée en ce que les esters d'acide acrylique ou méthacrylique sont des acrylates ou méthacrylates d'alkyle en C₁-C₂₀.

6. Composition selon l'une quelconque des revendications précédentes, caractérisée en ce que le polymère anionique non réticulé est choisi parmi :
- les copolymères comprenant (i) des acides ou anhydrides maléique, fumarique, itaconique , (ii) des esters vinyliques, des éthers vinyliques, des halogénures vinyliques, des dérivés phénylvinyliques, l'acide acrylique et ses esters :
- les copolymères d'acide méthacrylique et d'acrylate d'alkyle en C₁-C₆,
- les copolymères d'acide acrylique ou méthacrylique, d'acrylate ou de méthacrylate d'alkyle et de N-tertiobutylacrylamide, les terpolymères de vinylpyrrolidone/acide acrylique/méthacrylate de lauryle, les terpolymères d'acide méthacrylique, d'acrylate d'éthyle et d'allyl éther de stéaryl polyoxyéthyléné à 10 moles d'oxyde d'éthyléne.

7. Composition selon la revendication précédente, caractérisée en ce que le polymère anionique non réticulé est choisi parmi :
- les copolymères méthylvinyléther/anhydride maléique mono estérifié par des alcools en C₁-C₆.
- les copolymères d'acide méthacrylique et d'acrylate d'éthyle,
- les terpolymères de vinylpyrrolidone/acide acrylique/méthacrylate de lauryle.

8. Composition selon l'une quelconque des revendications précédentes, caractérisée par le fait que les gommes de guar non ioniques sont présentes dans des proportions comprises entre 0,01 % et 5 % en poids par rapport au poids total de la composition, et de préférence entre 0,1 et 2,5 %.

9. Composition selon l'une quelconque des revendications 1 à 8, caractérisée par le fait que les polymères anioniques non réticulés sont présents dans des proportions comprises entre 0,01% et 8 % en poids par rapport au poids total de la composition, et de préférence entre 0,1 et 3 %.

10. Composition selon l'une quelconque des revendications précédentes, caractérisée en ce que le rapport pondéral gomme de guar non ionique/polymère anionique non réticulé est inférieur à 1.

11. Composition selon l'une quelconque des revendications précédentes, caractérisée par le fait que le milieu cosmétiquement acceptable est constitué par de l'eau, un mélange d'eau et de solvants cosmétiquement acceptables choisis parmi les monoalcools, les polyalcools, les éthers de glycol ou les esters d'acides gras, utilisés seuls ou en mélange.

12. Composition selon l'une quelconque des revendications précédentes, caractérisée par le fait qu'elle contient également des agents tensio-actifs, des agents épaississants, des agents conservateurs, des séquestrants, des adoucissants, des parfums, des colorants, des agents modificateurs de viscosité, des agents modificateurs de mousse, des agents moussants, des stabilisateurs de mousse, des agents nacrants, des agents hydratants, des agents antipelliculaires, des agents antiséborrhéiques, des filtres solaires, des protéines, des vitamines, des plastifiants, des hydroxyacides, des électrolytes, des agents propulseurs, des parfums, et des agents conditionneurs.

13. Composition selon l'une quelconque des revendications précédentes, caractérisée par le fait qu'elle se présente sous forme de gel, de lait, de crème, de gel-crème, de spray, de lotion plus ou moins épaisse ou de mousse.

14. Procédé de traitement cosmétique non avant des matières kératiniques, caractérisé par le fait que l'on applique sur des matières kératiniques une composition telle que définie à l'une quelconque des revendications 1 à 13, et que l' on procède éventuellement à un rinçage après un éventuel temps de pose.

15. Procédé selon la revendication 14, caractérisé par le fait les matières kératiniques sont choisies parmi les cheveux et les cils.

## Claims

1. Non-washing cosmetic composition, characterized in that it contains, in a cosmetically acceptable medium, at least one nonionic guar gum and at least one non-crosslinked anionic polymer chosen from:
(A) - Copolymers comprising (i) one or more maleic, fumaric or itaconic acids or anhydrides and (ii) at least one monomer chosen from vinyl esters, vinyl ethers, vinyl halides, phenylvinyl derivatives, and acrylic acid and esters thereof;
- Copolymers comprising (i) one or more maleic, citraconic or itaconic anhydrides and (ii) one or more monomers chosen from allylic or methallylic esters optionally containing one or more acrylamide, methacrylamide or α-olefin group, acrylic or methacrylic esters, acrylic acid or methacrylic acid or vinylpyrrolidone in their chain,
the anhydride functions of these copolymers optionally being monoesterified or monoamidated.
(B) - Copolymers of acrylic acid or of methacrylic acid with one or more monoethylenic monomers such as ethylene, styrene, vinyl esters, acrylic acid esters or methacrylic acid esters.
(C) - Copolymers comprising (i) one or more acrylic or methacrylic acids, (ii) one or more monoethylenic monomers such as ethylene, styrene, vinyl esters, acrylic acid esters or methacrylic acid esters and (iii) one or more monomers chosen from vinyllactams, optionally polyoxyalkylenated C6-C22 alkyl allyl or methallyl ethers, vinyl acetate and N-alkyl(C3-C10)acrylamides.

2. Composition according to Claim 1, characterized in that the nonionic guar gum is modified with C₁-C₆ hydroxyalkyl groups.

3. Composition according to Claim 2, characterized in that the hydroxyalkyl groups are chosen from hydroxymethyl, hydroxyethyl, hydroxypropyl and hydroxybutyl groups.

4. Composition according to either of Claims 2 and 3, characterized in that the nonionic guar gum has a degree of hydroxyalkylation ranging between 0.4 and 1.2.

5. Composition according to any one of the preceding claims, characterized in that the acrylic or methacrylic acid esters are C₁-C₂₀ alkyl acrylates or methacrylates.

6. Composition according to any one of the preceding claims, characterized in that the non-crosslinked anionic polymer is chosen from:
- copolymers comprising (i) maleic, fumaric or itaconic acids or anhydrides, (ii) vinyl esters, vinyl ethers, vinyl halides, phenylvinyl derivatives, and acrylic acid and esters thereof;
- copolymers of methacrylic acid and of C₁-C₆ alkyl acrylate,
- copolymers of acrylic or methacrylic acid, of alkyl acrylate or methacrylate and of N-tert-butylacrylamide, terpolymers of vinylpyrrolidone/acrylic acid/lauryl methacrylate, and terpolymers of methacrylic acid, of ethyl acrylate and of stearyl allyl ether polyoxyethylenated with 10 mol of ethylene oxide.

7. Composition according to the preceding claim, characterized in that the non-crosslinked anionic polymer is chosen from:
- copolymers of methyl vinyl ether/maleic anhydride monoesterified with C₁-C₆ alcohols,
- copolymers of methacrylic acid and of ethyl acrylate,
- terpolymers of vinylpyrrolidone/acrylic acid/lauryl methacrylate.

8. Composition according to any one of the preceding claims, characterized in that the nonionic guar gums are present in proportions of between 0.01 % and 5 % by weight relative to the total weight of the composition, and preferably between 0.1 and 2.5 %.

9. Composition according to any one of Claims 1 to 8, characterized in that the non-crosslinked anionic polymers are present in proportions of between 0.01 % and 8 % by weight approximately relative to the total weight of the composition, and preferably between 0.1 and 3 %.

10. Composition according to any one of the preceding claims, characterized in that the nonionic guar gum/non-crosslinked anionic polymer weight ratio is less than 1.

11. Composition according to any one of the preceding claims, characterized in that the cosmetically acceptable medium consists of water, a mixture of water and cosmetically acceptable solvents chosen from monoalcohols, polyalcohols, glycol ethers or fatty acid esters, used alone or as a mixture.

12. Composition according to any one of the preceding claims, characterized in that it also contains surfactants, thickeners, preserving agents, sequestering agents, softeners, fragrances, dyes, viscosity modifiers, foam modifiers, foaming agents, foam stabilizers, pearlescent agents, moisturizing agents, antidandruff agents, antiseborrhoeic agents, sunscreens, proteins, vitamins, plasticizers, hydroxy acids, electrolytes, propellants, fragrances and conditioners.

13. Composition according to any one of the preceding claims, characterized in that it is in the form of a gel, a milk, a cream, a cream-gel, a spray, a relatively thickened lotion or a mousse.

14. Process for the non-washing cosmetic treatment of keratin substances, characterized in that a composition as defined in any one of Claims 1 to 13 is applied to keratin substances, possibly followed by a rinsing operation after an optional period of leaving the applied composition in place.

15. Process according to Claim 14, characterized in that the keratin substances are chosen from the hair and the eyelashes.

## Patentansprüche

1. Nicht reinigende kosmetische Zusammensetzung, dadurch gekennzeichnet, daß sie in einem kosmetisch akzeptablen Medium mindestens ein nichtionisches Guargummi und mindestens ein nicht vernetztes anionisches Polymer enthält, das ausgewählt ist unter:
(A) - den Copolymeren, die (i) Maleinsäure, Fumarsäure, Itaconsäure, Maleinsäureanhydrid, Fumarsäureanhydrid und/oder Itaconsäureanhydrid und (ii) mindestens ein Monomer enthalten, das unter Vinylestern, Vinylethern, Vinylhalogeniden, Phenylvinylderivaten, Acrylsäure und ihren Estern ausgewählt ist;
- den Copolymeren, die (i) Maleinsäureanhydrid, Citraconsäureanhydrid und/oder Itaconsäureanhydrid und (ii) ein Monomer oder mehrere Monomere enthalten, die unter Allylestern oder Methallylestern ausgewählt sind, die in ihrer Kette gegebenenfalls eine oder mehrere Acrylamid-, Methacrylamid-, α-Olefin-, Acrylester-, Methacrylester-, Acrylsäure-, Methacrylsäure- oder Vinylpyrrolidongruppen aufweisen,
wobei die Anhydridgruppen dieser Copolymere gegebenenfalls einfach verestert oder einfach amidiert sind.
(B) - den Copolymeren von Acrylsäure oder Methacrylsäure mit einem oder mehreren Monomeren mit einer ethylenisch ungesättigten Bindung, wie Ethylen, Styrol, Vinylestern, Acrylsäureestern oder Methacrylsäureestern.
(C) - den Copolymeren, die (i) Acrylsäure und/oder Methacrylsäure, (ii) ein oder mehrere Monomere mit einer ethylenisch ungesättigten Bindung, wie Ethylen, Styrol, Vinylester, Acrylsäureester oder Methacrylsäureester und (iii) ein Monomer oder mehrere Monomere enthalten, die unter Vinyllactamen, C₆₋₂₂-Alkylallylethern oder C₆₋₂₂-Alkylmethallylethern, die gegebenenfalls polyoxyalkyliert sind, Vinylacetat und N-C₃₋₁₀-Alkylacrylamiden ausgewählt sind.

2. Zusammensetzung nach Anspruch 1, dadurch gekennzeichnet, daß das nichtionische Guargummi mit C₁₋₆-Hydroxyalkylgruppen modifiziert ist.

3. Zusammensetzung nach Anspruch 2, dadurch gekennzeichnet, daß die Hydroxyalkylgruppen unter Hydroxymethyl-, Hydroxyethyl-, Hydroxypropyl- und Hydroxybutylgruppen ausgewählt sind.

4. Zusammensetzung nach einem der Ansprüche 2 und 3, dadurch gekennzeichnet, das das nichtionische Guargummi einen Hydroxyalkylierungsgrad aufweist, der im Bereich von 0,4 bis 1,2 liegt.

5. Zusammensetzung nach einem der vorhergehenden Ansprüche, dadurch gekennzeichnet, daß die Acrylsäureester oder Methacrylsäureester C₁₋₂₀-Alkylacrylate oder C₁₋₂₀-Alkylmethacrylate sind.

6. Zusammensetzung nach einem der vorhergehenden Ansprüche, dadurch gekennzeichnet, daß das nicht vernetzte anionische Polymer ausgewählt ist unter:
- den Copolymeren, die (i) Maleinsäure, Fumarsäure, Itaconsäure, Maleinsäureanhydrid, Fumarsäureanhydrid oder Itaconsäureanhydrid und (ii) Vinylester, Vinylether, Vinylhalogenide, Phenylvinylderivate, Acrylsäure und ihre Ester enthalten,
- den Copolymeren von Methacrylsäure und C₁₋₆-Alkylacrylat,
- den Copolymeren von Acrylsäure oder Methacrylsäure, Alkylacrylat oder Alkylmethacrylat und N-tert.-Butylacrylamid, den Vinylpyrrolidon/Acrylsäure/Laurylmethacrylat-Terpolymeren und den Terpolymeren von Methacrylsäure, Ethylacrylat und mit 10 Mol Ethylenoxid polyethoxyliertem Stearylallylether.

7. Zusammensetzung nach dem vorhergehenden Anspruch, dadurch gekennzeichnet, daß das nicht vernetzte anionische Polymer ausgewählt ist unter:
- den Copolymeren von Methylvinylether und mit C₁₋₆-Alkoholen einfach verestertem Maleinsäureanhydrid,
- den Copolymeren von Methacrylsäure und Ethylacrylat und
- den Vinylpyrrolidon/Acrylsäure/Laurylmethacrylat-Terpolymeren.

8. Zusammensetzung nach einem der vorhergehenden Ansprüche, dadurch gekennzeichnet, daß die nichtionischen Guargummen in Mengenanteilen im Bereich von 0,01 bis 5 Gew.-%, bezogen auf das Gesamtgewicht der Zusammensetzung, und vorzugsweise im Bereich von 0,1 bis 2,5 Gew.-% vorliegen.

9. Zusammensetzung nach einem der Ansprüche 1 bis 8, dadurch gekennzeichnet, daß die nicht vernetzten anionischen Polymere in Mengenanteilen im Bereich von 0,01 bis 8 Gew.-%, bezogen auf das Gesamtgewicht der Zusammensetzung, und vorzugsweise im Bereich von 0,1 bis 3 Gew.-% vorliegen.

10. Zusammensetzung nach einem der vorhergehenden Ansprüche, dadurch gekennzeichnet, daß das Gewichtsverhältnis nichtionisches Guargummi/nicht vernetztes anionisches Polymer unter 1 liegt.

11. Zusammensetzung nach einem der vorhergehenden Ansprüche, dadurch gekennzeichnet, daß das kosmetisch akzeptable Medium aus Wasser oder einem Gemisch von Wasser und kosmetisch akzeptablen Lösemitteln besteht, die unter einwertigen Alkoholen, mehrwertigen Alkoholen, Glykolethern und Fettsäureestern ausgewählt sind, die allein oder im Gemisch verwendet werden können.

12. Zusammensetzung nach einem der vorhergehenden Ansprüche, dadurch gekennzeichnet, daß sie ferner grenzflächenaktive Stoffe, Verdickungsmittel, Konservierungsmittel, Maskierungsmittel, reizlindernde Mittel, Parfums, Färbemittel, Mittel zum Modifizieren der Viskosität, Schaummodifikatoren, Schaumbildner, Schaumstabilisatoren, Perlglanzmittel, Hydratisierungsmittel, Mittel gegen Schuppen, Mittel gegen Seborrhoe, Sonnenschutzfilter, Proteine, Vitamine, Weichmacher, Hydroxysäuren, Elektrolyte, Treibmittel, Parfums oder Konditioniermittel enthält.

13. Zusammensetzung nach einem der vorhergehenden Ansprüche, dadurch gekennzeichnet, daß sie als Gel, Milch, Creme, Gelcreme, Spray, mehr oder weniger dickflüssige Lotion oder als Schaum vorliegt.

14. Verfahren zur nicht reinigenden kosmetischen Behandlung von Keratinsubstanzen, dadurch gekennzeichnet, daß eine in einem der Ansprüche 1 bis 13 definierte Zusammensetzung auf die Keratinsubstanzen aufgetragen wird und daß anschließend gegebenenfalls, eventuell nach einer Einwirkungszeit, gespült wird.

15. Verfahren nach Anspruch 14, dadurch gekennzeichnet, daß die Keratinsubstanzen unter den Haaren und den Wimpern ausgewählt sind.
